# EUROPÄISCHE PATENTSCHRIFT

(11) **EP 1 584 319 B1**
(45) Veröffentlichungstag und Bekanntmachung des Hinweises auf die Patenterteilung: **25.06.2008**
(21) Anmeldenummer: 05005831.2
(22) Anmeldetag: 17.03.2005
(51) Int. Cl.: A61K 6/083, C03B 19/10

(54) **Agglomerierte Füllstoffe für Dentalmaterialien**
Agglomerated filler for dental materials
Charge agglomérée pour matèriaux dentaires

(30) Priorität: 07.04.2004 DE 102004017562
(43) Veröffentlichungstag der Anmeldung: 12.10.2005
(73) Patentinhaber: Heraeus Kulzer GmbH, 63450 Hanau (DE)
(72) Erfinder: Ruppert, Klaus, Dr., 63477 Maintal (DE); Erdrich, Albert, Dr., 61231 Bad Nauheim (DE); Grundler, Andreas, Dr., 42117 Wuppertal (DE)
(74) Vertreter: Kühn, Hans-Christian

(56) Entgegenhaltungen:
- EP-A- 0 113 926
- WO-A-81/01366
- US-A- 4 126 422
- US-A- 6 020 395

## Beschreibung

Die Erfindung betrifft agglomerierte anorganische Füllstoffe für Dentalmaterialien.

Zähne und demzufolge auch ihre Füllungen sind - besonders beim Zähneputzen - Abrasionsprozessen ausgesetzt. Bei den bekannten Komposit-Zahnfüllungsmaterialien aus organischer und verstärkenden Füllstoffen findet die Abrasion in der Regel in mehreren Schritten statt:

Zunächst wird durch ein abrasives Medium, z.B. Zahnpasta oder Steinzellen im Speisebrei, die Matrix abgetragen, welche die Füllstoff-Partikel einhüllt. Die Matrix ist in der Regel ein Polymermaterial, z.B. ein vernetztes Polymer auf Basis Methacrylsäureester.

Es kommt ein Zeitpunkt, an dem die Füllstoffpartikel soweit aus der Oberfläche des Polymeren herausgearbeitet sind, dass sie ihren Halt verlieren und aus der Oberfläche herausbrechen.

Zurück bleiben Vertiefungen in der Oberfläche (Krater).

Sowohl die erhabenen Füllstoffpartikel als auch die Krater führen zu diffuser Lichtreflexion in der Mikrorauhigkeit der Oberfläche, verringern den Anteil total reflektierten Lichts und vermindern darüber den oberflächlichen Glanz der ursprünglich perfekt polierten Oberfläche. Dieser partielle Abrasionsprozess ist daher aus ästhetischen Gründen unerwünscht.

Der Grund für die selektive Abrasion liegt in der wesentlich höheren Härte und Abrasionsresistenz der Füllstoffpartikel im Vergleich zur einbettenden und umfassenden Polymermatrix. Zur Vermeidung des partiellen oder selektiven Abrasionsprozesses wurde ein Konzept entwickelt, das im Wesentlichen darauf beruht, dass die Partikel nicht mehr aus der Oberfläche hervorstehen sollen. Dazu werden agglomerierte Füllerpartikel zur Verfügung gestellt, die aus Unterpartikeln mit Teilchengrößen im Mikrometer oder Submikron-Bereich bestehen, deren Härte und Agglomerationsfestigkeit der des umschließenden Polymersystems ähnlicher ist. Es wurden bereits Füllmaterialen entwickelt, die dieses Prinzip ausnutzen und agglomerierte Cluster aus Nanoteilchen enthalten: Ein Zahnfüllungsmaterial, das solche agglomerierten Füllstoffe enthält, ist das sog. "FiItek™ Supreme Universal Restorative" der Firma 3M™ ESPE™.

Es besteht im Wesentlichen aus einem Polymeranteil mit den Komponenten BIS-GMA, BIS-EMA, UDMA und geringen Mengen TEGDMA sowie aus Füllstoffen und wird in verschiedenen Farbabstufungen (sog. Shades) geliefert:

Die transluzenten, nicht-röntgenopaken Teile des Materialsortiments enthalten eine Kombination von nicht-agglomeriertem/nicht-aggregiertem 75 nm Kieselsäure-Nanofüllstoff sowie locker gebundene, agglomerierte Kieselsäure-Nanocluster bestehend aus Agglomeraten von Kieselsäure-Nano-Primärteilchen (75 nm Partikelgröße). Der Größenbereich für die Agglomerate, die auch als Cluster bezeichnet werden, liegt bei 0,6 bis 1,4 Mikrometer. Der Füllgrad beträgt 72,5 Gew.-%.

Die nicht-transluzenten, röntgenopaken Teile des Sortiments enthalten eine Kombination nichtagglomerierter/nicht-aggregierter 20 nm Kieselsäure-Nanofüller sowie locker gebundene agglomerierte Zirkonoxid/Kieselsäure-Nanocluster, die Agglomerate von ZrO₂/SiO₂ Primärteilchen von mit Teilchengrößen von 5 - 20 nm sind. Die Clustergröße liegt wieder bei 0,6 bis 1,4 Mikrometer. Der Füllgrad beträgt 78,5 Gew-%. (Produktprofil Filtek™ Supreme).

Die Cluster werden durch thermische Behandlung erhalten (z.B. WO 200130306A1, S. 31 und WO 200130304A1, S.7).

Aufgabe der Erfindung ist es, weitere agglomerierte anorganische Füllungsmaterialien zur Verfügung zu stellen. Diese Agglomerate weisen eine so hohe mechanische Stabilität auf, dass sie den mechanischen Belastungen beim Herstellungsprozess standzuhalten, beim Abrasionsprozess aber nicht zur Gänze, sondern nur teil- bzw. schichtweise aus dem fertigen Komposit durch selektive Abrasion herausgearbeitet werden.

Die Aufgabe wird dadurch gelöst, dass Primärpartikel aus Glas durch thermische Behandlung zu Agglomeraten zusammengebacken werden, d.h. sie schmelzen oberflächlich mit mindestens einem ihrer Nachbarn zusammen. Es ergeben sich agglomerierte anorganische Glas-Füllstoffe für Dentalmaterialien bestehend aus 0,5 bis 50 µm großen Agglomeraten von 200 bis 7000 nm großen anorganischen Glas-Partikeln, die an ihren Grenzflächen mit mindestens einem benachbarten Partikel verschmolzen sind.

Die Partikelgröße definiert sich über den sog. d 50-Wert.

Die Erfindung betrifft somit Füllstoffe nach Patentanspruch 1, Verfahren zu deren Herstellung (Ansprüche 2-4) sowie ihre Verwendung in Dentalmaterien (Ansprüche 5 - 9).

Die Dentalmaterialien können noch weitere anorganische Füllstoffe enthalten, z.B. mit Partikelgrößen von 2 bis 30 nm und von 30 bis 200 nm. Dazu zählen anorganische Oxide wie SiO₂, Al₂O₃, ZrO₂, Y₂O₃, insbesondere Fällungskieselsäuren und Nanofüller, wie z.B. in US 5,936,006 beschrieben. Die agglomerierten Gläser und die weiteren Füllstoffe können oberflächenmodifiziert sein, insbesondere silanisiert, z.B. durch Behandlung mit gamma-Methacryloxypropyltrimethoxysilan.

Das Zusammenbacken erfolgt durch gesteuerte thermische Behandlung. Resultat ist das Aneinanderschmelzen der Partikel an den Grenzflächen. Die Steuerung der Behandlungszeit und -temperatur hat das Ziel, dass die Festigkeit so hoch ist, dass die Partikel den Herstellungsprozess der Dentalmaterialien überstehen, aber so niedrig, dass die Partikel bei der Abrasion nicht zur Gänze, sondern schichtweise bzw. teilweise beim Abrasionsprozess mit abgetragen werden. Das sorgt später für eine stets mikroskopisch glatte Oberfläche mit dauerhaft zufriedenstellendem Glanz.

Das zusammengebackene Material wird zweckmäßig zu 0,5 bis 50 µm großen Teilchen zerkleinert, vorzugsweise durch Mahlverfahren, ggf. mit anschließendem Sieb- oder Sichtverfahren.

Die Erfindung betrifft somit auch ein Verfahren zur Herstellung von agglomerierten anorganischen Füllungsmaterialien, mit den Schritten
A Bereitstellen von 200 m bis 7000 nm großen anorganischen Partikeln, insbesondere Glas-Partikeln durch Zerkleinern großer Partikel,
B thermische Behandlung unter Anschmelzen bei 200 bis 1300 °C (Calcinieren der Glaspartikel),
C ggf. schnelles Abkühlen,
D Zerkleinern des thermisch zusammengebackenen Materials.

Als Glasmaterial kommen bevorzugt Dentalgläser in Frage, besonders solche, die chemische Elemente aus der Gruppe Ba, Al, Si, O, F, B, Sr, Zr enthalten, wie z.B. Ba-, Sr-, Ca-, Li-A1-Silikatgläser oder deren Mischungen, besonders Li-Al-Borosilikatgläser oder deren Mischungen sowie Bariumaluminumborosilikatglas.

Die Temperaturen bei der thermischen Behandlung hängen vom Material ab und liegen im Allgemeinen im Bereich von 200 bis 1300 °C.

Ausgangsmaterial kann eine Dispersion sein, wie z.B. in US 4,503,169 beschrieben, wobei allerdings zerkleinerte Glaspartikel verwendet werden. Die thermische Behandlung der Teilchen kann auf verschiedene Weise erfolgten, z.B. direkt in einer Flamme oder im heißen Gasstrom (gemäß US 5,559,170, Spalten 15, 16, EP 757 664, Anspruch 29) oder durch Sprühtrocken einer Dispersion und anschließendes Calcinieren (vgl. US 6,362,251 B1, Beispiele 1 - 4).

In Frage kommen auch Glas-Granulate, die analog der Methode der DE 44 24 044 durch Verdichten einer Suspension und anschließendes Calcinieren/Teil-Verschmelzen hergestellt werden.

Ein weiteres mögliches Verfahren ist das Heißpressen nach DE 198 21 679 A1. Dabei werden bevorzugt Partikel zweier verschiedener Gläser eingesetzt, deren eines bei geringeren Temperaturen erweicht als das andere. Das niedrig "schmelzende" Glas erzeugt dann ein Lot für das höher "schmelzende".

Ebenso kann auch eine Feinst-Fraktion aus dem gleichen Material eingesetzt werden, auch hier sollte es aufgrund der höheren Sinteraktivität zu niedrigeren Schmelztemperaturen kommen.

Möglich ist es auch, ein Verfahren nach DE 101 63 179 so zu modifizieren, dass statt der pyrogenen Kieselsäuren feingemahlene Gläser eingesetzt werden, oder es werden gemäß DE 196 29 690 C2 und DE 196 0 2525 A1 bzw. US 5,858,325 Suspensionen/Schlicker aus Glasteilchen und einem Lösungsmittel durch (Wirbelschicht-)Sprühgranulation granuliert und dann einer Schocksinterung unterzogen.

Nach der thermischen Behandlung werden die agglomerierten Partikel zweckmäßigerweise schnell gekühlt, um ein zu starkes Zusammenbacken zu verhindern.

### Herstellungsbeispiel:

Bariumaluminumsilikatglas wird in einer Mühle feingemahlen und gesiebt. Die Fraktion von ca. 200 bis 500 nm Partikelgröße wird weiterverarbeitet.

Durch Mischen mit Wasser in einem Schnellmischer wird eine Suspension hergestellt, die nach Absetzen ein fließfähiges Granulat ergibt. Das Granulat wird thermisch bei 650 bis 950°C behandelt, schnell abgekühlt und anschließend zerkleinert.

Es ergeben sich Agglomeratteilchen mit Durchmessern von 2 bis 15 µm.

## Patentansprüche

1. Agglomerierte anorganische Füllstoffe für Dentalmaterialien bestehend aus 0,5 bis 50 µm großen Agglomeraten von 200 bis 7000 nm großen anorganischen Partikeln, die an ihren Grenzflächen mit mindestens einem benachbarten Partikel verschmolzen sind.

2. Verfahren zur Herstellung von Füllstoffen nach Anspruch 1, mit den Schritten
A Bereitstellen von 200 bis 7000 nm großen anorganischen Partikeln durch Zerkleinern größerer Partikel,
B thermische Behandlung unter Anschmelzen bei 200 bis 1300 °C,
C ggf. schnelles Abkühlen,
D Zerkleinern des thermisch zusammengebackenen Materials.

3. Verfahren nach Anspruch 2, wobei in Schritt A das Bereitstellen durch Mahlen von Glas erfolgt.

4. Verfahren nach Anspruch 3, wobei das Glas chemische Elemente aus der Gruppe Ba, Al, Si, O, F, B, Sr, Zr enthält.

5. Verwendung von Füllstoffen nach Anspruch 1 zur Herstellung von Dentalmaterial.

6. Dentalmaterial enthaltend einen oder mehrere Füllstoffe nach Anspruch 1.

7. Dentalmaterial nach Anspruch 6, enthaltend zusätzlich einen oder mehrere anorganische Füllstoffe mit Teilchengrößen von 2 bis 30 nm und/oder 30 bis 200 nm.

8. Dentalmaterial nach Anspruch 6 oder 7, wobei die Füllstoffe oberflächenmodifiziert sind.

9. Dentalmaterial nach Anspruch 8, wobei die Füllstoffe silanisiert sind.

## Claims

1. Agglomerated inorganic fillers for dental materials consisting of 0.5 to 50 µm agglomerates of 200 to 7000 nm inorganic particles, which are fused at their interfaces to at least one adjacent particle.

2. Method for the production of fillers according to Claim 1, comprising the steps
A provision of 200 to 7000 nm inorganic particles by comminution of larger particles,
B thermal treatment with partial melting at 200 to 1300°C
C optionally rapid cooling,
D comminution of the thermally agglomerated material.

3. Method according to Claim 2, wherein, in step A, the provision is effected by milling of glass.

4. Method according to Claim 3, wherein the glass contains chemical elements from the group consisting of Ba, Al, Si, O, F, B, Sr and Zr.

5. Use of fillers according to Claim 1 for the production of dental material.

6. Dental material containing one or more fillers according to Claim 1.

7. Dental material according to Claim 6, additionally containing one or more inorganic fillers having particle sizes of 2 to 30 nm and/or 30 to 200 nm.

8. Dental material according to Claim 6 or 7, wherein the fillers are surface-modified.

9. Dental material according to Claim 8, wherein the fillers are silanized.

## Revendications

1. Agents de charge inorganiques agglomérés pour matériaux dentaires composés d'agglomérats de grosseur comprise entre 0,5 et 50 µm de particules inorganiques de grosseur comprise entre 200 et 7 000 nm, lesquelles sont réunies à au moins une particule adjacente par fusion de leurs surfaces limitrophes.

2. Procédé de fabrication d'agents de charge selon la revendication 1, comprenant les étapes de :
A préparation de particules inorganiques de grosseur comprise entre 200 et 7 000 nm par fragmentation de particules de grosseur supérieure,
B traitement thermique par fusion à température comprise entre 200 et 1300 °C,
C refroidissement rapide le cas échéant,
D fragmentation de la matière thermofusionnée.

3. Procédé selon la revendication 2, où la préparation de l'étape A est effectuée par broyage de verre.

4. Procédé selon la revendication 3, où le verre contient des éléments chimiques du groupe Ba, A1, Si, O, F, B, Sr, Zr.

5. Utilisation d'agents de charge selon la revendication 1 pour la fabrication de matériau dentaire.

6. Matériau dentaire contenant un ou plusieurs agents de charge selon la revendication 1.

7. Matériau dentaire selon la revendication 6, contenant en outre un ou plusieurs agents de charge inorganiques avec des grosseurs de particules comprises entre 2 et 30 nm et/ou 30 et 200 nm.

8. Matériau dentaire selon la revendication 6 ou 7, où les agents de charge ont subi une modification superficielle.

9. Matériau dentaire selon la revendication 8, où les agents de charge sont silanisés.
